# EUROPÄISCHE PATENTSCHRIFT

(11) **EP 1 631 246 B1**
(45) Veröffentlichungstag und Bekanntmachung des Hinweises auf die Patenterteilung: **25.07.2007**
(21) Anmeldenummer: 03732440.7
(22) Anmeldetag: 22.05.2003
(51) Int. Cl.: A61K 8/29, A61K 8/49, A61K 8/60, A61K 8/73, A61K 8/34, A61K 8/06, A61Q 17/04

(54) **MISCHUNG AUS TITANDIOXID UND METHYLEN-BIS-BENZOTRIAZOLYLPHENOL**
TITANIUM DIOXIDE AND METHYLENE-BIS-BENZOTRIAZOLYL-PHENOL MIXTURE
MELANGE DE DIOXYDE DE TITANE ET DE METHYLENE-BIS-BENZOTRIAZOLYLE-PHENOL

(43) Veröffentlichungstag der Anmeldung: 08.03.2006
(73) Patentinhaber: KEMIRA PIGMENTS OY, 28840 Pori (FI)
(72) Erfinder: DAHMS, Gerd, 47138 Duisburg (DE); SEIDEL, Holger, 47055 Duisburg (DE); ANTINLUOMA, Olli-Pekka, FIN-28120 Pori (FI); MULTISILTA, Riku, FIN-28130 Pori (FI)
(74) Vertreter: Peterreins, Frank
(86) Internationale Anmeldenummer: PCT/EP2003/005368
(87) Internationale Veröffentlichungsnummer: WO 2004/103330

(56) Entgegenhaltungen:
- EP-A- 1 068 866
- EP-A- 1 093 799
- EP-A- 1 281 390
- WO-A-03/039507
- WO-A-03/063814
- DE-A- 10 154 547
- DE-A- 10 155 716
- DE-A- 19 923 645
- "THE USE OF MIXTURES OF THE UVA-ABSORBER METHYLENE BIS-BENZOTRIAZOLYL TETRAMETHYLBUTYLPHENOL WITH UVB-ABSORBERS IN SUNSCREEN FORMULATIONS" RESEARCH DISCLOSURE, KENNETH MASON PUBLICATIONS, HAMPSHIRE, GB, Nr. 449, September 2001 (2001-09), Seiten 1478-1484, XP001128240 ISSN: 0374-4353

## Beschreibung

Die Erfindung betrifft Mischungen aus, vorzugsweise teilchenförmigem, Titandioxid und einer organischen Mikropigment-Dispersion auf Basis von Methylen-bis-benzotriazolylphenol, die Verwendung derartiger Mischungen zur Herstellung von kosmetischen OW-, WO-, PO- oder multiplen Emulsionen, Verfahren zu deren Herstellung und so erhaltene WO-Emulsionen. Es handelt sich dabei insbesondere um Lichtschutzmittel-Emulsionen.

Die schädigende Wirkung des ultravioletten Teils der Sonnenstrahlung auf die menschliche Haut ist allgemein bekannt. Daher werden seit längerer Zeit Lichtschutzmittel bereit gestellt, die UV-Strahlung im UVA- und UVB-Bereich absorbieren.

Lichtschutzmittel enthalten heutzutage in der Regel organische und gegebenenfalls zusätzlich anorganische Lichtschutzfilter. Ein UV-Filter mit vorteilhaften Eigenschaften ist das 2,2'-Methylen-bis-(6-(2H-benzotriazol-2-yl)-4-(1,1,3,3-tetramethylbutyl)phenol). Diese Verbindung wird von Ciba Specialty Chemicals unter der Bezeichnung Tinosorb^{®} M als eine 50 Gew.-%-ige wässrige Dispersion vertrieben. Die Dispersion erscheint als weiße Flüssigkeit, die Absorptionsmaxima bei 302 und 357 nm zeigt. Die Dispersion enthält 50 Gew.-% des Methylen-bis-benzotriazolyl-tetramethylbutylphenols, 7,5 Gew.-% Decylglucosid als Emulgator, 0,2 Gew.-% Xanthan Gumen als Verdicker und 0,4 Gew.-% Propylenglykol, sowie als Rest Wasser. Das organische Pigment liegt dabei in Form von mikrofeinen Teilchen vor. Der UV-Absorber wirkt damit sowohl als Mikropigment wie auch als organischer UV-Absorber. Als Sonnenschutzfilter wirkt er dreifach durch UV-Absorption durch ein photostabiles organisches Molekül, durch Lichtstreuung und durch Lichtreflektion aufgrund der mikrofeinen Struktur. Es ist bekannt, dass Tinosorb^{®} M in O/W-Emulsionen eingebracht werden kann, nicht jedoch in W/O-Emulsionen.

Auch das Einbringen in O/W-Emulsionen bereitet Schwierigkeiten und unterliegt Beschränkungen. Die DE-A-197 26 184 beschreibt die Formulierung von Tinosorb^{®} M in OW-Emulsionen und OWO-Emulsionen, wobei spezifische Emulgatoren eingesetzt werden. Es ist angegeben, dass die Zubereitungen außerdem anorganische Pigmente, beispielsweise auf Basis von Titandioxyd enthalten können. Es ist ferner angegeben, dass die anorganischen Pigmente in Form von Mikropigmenten vorliegen können. Es werden nur allgemein Einsatzmengen von 0,1 bis 30 Gew.-%, vorzugsweise 0,5 bis 10 Gew.-%, insbesondere 1 bis 6 Gew.-%, bezogen auf das Gesamtgewicht der Zubereitungen, genannt. Es wird keine spezielle Reihenfolge bei der Formulierung der OW-Emulsionen genannt. Insbesondere ist keine Formulierung eines Gemisches aus teilchenförmigem Titandioxid und Tinosorb^{®} M genannt.

Ähnliche OW-Emulsionen, die beispielsweise 6 Gew.-% Tinosorb^{®} M und 2 Gew.-% Titandioxid enthalten können, sind in der DE-A-199 23 645 erwähnt. Es werden jedoch keine spezifischen Kombinationen von Tinosorb^{®} M und Titandioxid aufgeführt. Die Anmeldung zielt insbesondere auf die Mitverwendung von Bornitrid zur Verbesserung des Hautgefühls von Zubereitungen ab.

DE-A-199 23 713 betrifft ähnliche Systeme, die zudem Flavonderivate und/oder Flavanonderivate aufweisen.

Ein häufig auftretendes Problem beim Einsatz von Tinosorb^{®} M in kosmetischen Formulierungen ist die Erreichung eines ausreichenden Dispersionsgrades, damit der Wirkstoff eine optimale Wirkung entfalten kann. Aus diesem Grund wird Tinosorb^{®} M als 50 Gew.-%-ige wässrige Dispersion eingesetzt, die einen hohen Gehalt an Decylglucosid als Emulgator bzw. Dispergator aufweist. Die große Menge an Dispergator ist notwendig aufgrund seiner nur teilweisen Wirksamkeit.

Als Folge dieser Formulierung des organischen Mikropigments ist die Herstellung von kosmetischen und anderen Formulierungen stark eingeschränkt, da der vorhandene Emulgator die Herstellung der Formulierungen beeinflusst bzw. beeinträchtigt. Es sind beispielsweise keine WO-Emulsionen herstellbar, und OW-Emulsionen sind nur bedingt und mit eingeschränkten Einsatzstoffen herstellbar.

Die Effizienz des organischen Mikropigments als UV-Filter hängt jedoch von der Art der Formulierung ab. Wenn aufgrund der ursprünglichen Formulierung des organischen Mikropigments mit dem hohen Dispergatorgehalt keine oder kaum Freiheitsgrade für eine Einstellung einer fertigen Formulierung bestehen, ist keine Effizienzoptimierung möglich.

Der Einsatz von Tinosorb^{®} M in WO-Formulierungen wäre besonders vorteilhaft, da WO-Formulierungen eine gute und schnelle Filmbildung erlauben und eine gute Wasserfestigkeit aufweisen. Tinosorb^{®} M zeigt dabei zudem keine Zersetzung unter Lichteinstrahlung und keine Penetration der Haut, wie sie bei flüssigen Lichtschutzfiltern auftritt.

Aufgabe der vorliegenden Erfindung ist die Bereitstellung einer Formulierung für organische Mikropigment-Dispersionen auf Basis von Methylen-bis-benzotriazolylphenol-Derivaten, die die Nachteile der bekannten Formulierungen vermeidet und ein einfaches Einbringen in eine Vielzahl von Formulierungen ermöglicht, so dass eine Optimierung von Formulierungen möglich wird.

Die Aufgabe wird erfindungsgemäß gelöst durch eine Mischung, bestehend aus
a) 10 bis 40 Gew.-%, teilchenförmigem Titandioxid mit einer mittleren Teilchengröße im Bereich von 5 bis 500 nm, auf das organische und/oder anorganische Hilfsmittel aufgebracht sein können, als Komponente A,
b) 60 bis 90 Gew.-% einer organischen Mikropigment-Dispersion als Komponente B, bestehend aus
   b1) 20 bis 75 Gew.-% mindestens eines Methylen-bis-benzotriazolylphenols, in dem die Benzotriazolylreste und Phenolreste jeweils unabhängig durch einen oder mehrere C₁ - ₂₀-Alkylreste, C₁ - ₂₀-Alkoxyreste, C₁ - ₂₀-Hydroxyalkylreste, C₆ - ₁₂-Arylreste, Halogen, Hydroxylgruppen oder Cyanogruppen substituiert sein können, als organisches Mikropigment B1,
   b2) 2 bis 15 Gew.-% mindestens eines Emulgators B2,
   b3) 0,5 bis 5 Gew.-% organischen Hilfsstoffen B3,
   b4) 22,5 bis 77,5 Gew.-% Wasser als Komponente B4,
wobei die Gesamtmenge der Komponenten B1 bis B4 100 Gew.-% ergibt,
wobei bis zu 20 Gew.-% der Mischung durch andere Inhaltsstoffe ersetzt sein können, erhältlich durch Zusatz der Komponente A zur Komponente B.

Es wurde erfindungsgemäß gefunden, dass durch Zusatz von teilchenförmigem Titandioxid mit einer Teilchengröße im Bereich von 5 bis 500 nm die Formulierbarkeit der organischen Mikropigment-Dispersionen erheblich verbessert wird. Beim Einsatz der erfindungsgemäßen Mischung können OW-, WO-, PO- oder multiple Emulsionen in einem weiten Bereich von Zusammensetzungen hergestellt und optimiert werden, ohne dass es zu Dispergierungsproblemen bezüglich des organischen Mikropigments kommt.

Der Zusatz des teilchenförmigen Titandioxids verbessert damit die Formulierbarkeit des genannten organischen Mikropigments ganz erheblich, so dass leistungsoptimierte Zusammensetzungen zugänglich werden. Die Optimierung bezieht sich dabei insbesondere auf die Lichtschutzwirkung in Lichtschutzmitteln bzw. Sonnenschutzmitteln, die auf die menschliche Haut aufgetragen werden.

Die erfindungsgemäße Mischung besteht aus den Komponenten A und B, wobei bis zu 20 Gew.-%, vorzugsweise bis zu 10 Gew.-%, insbesondere bis zu 5 Gew.-% der Mischung durch andere Inhaltsstoffe ersetzt sein können. Gemäß einer bevorzugten Ausführungsform besteht die Mischung nur aus den Komponenten A und B.

Komponente A liegt in den erfindungsgemäßen Mischungen in einer Menge von 5 bis 60 Gew.-%, vorzugsweise 10 bis 40 Gew.-%, insbesondere 15 bis 30 Gew.-% vor. Bei Komponente A handelt es sich um, vorzugsweise teilchenförmiges, Titandioxid, auf das organische und/oder anorganische Hilfsmittel aufgebracht sein können. Das, vorzugsweise teilchenförmige, Titandioxid weist dabei vorzugsweise eine mittlere Teilchengröße im Bereich von 5 bis 500nm, besonders bevorzugt 10 bis 80 nm auf. Insbesondere handelt es sich um mikrofeines Titandioxid. Das Titandioxid kann in den gängigen Kristallformen in unbehandelter oder oberflächenbehandelter Form eingesetzt werden. Eine Oberflächenbehandlung mit organischen und/oder anorganischen Hilfsmitteln ist möglich. Als organische Hilfsmittel kommen insbesondere organische Polymere in Betracht. Anorganische Hilfsmittel können Metalloxide und/oder Nichtmetalloxide sein.

Besonders bevorzugt liegt in Komponente A nur nicht oberflächenbehandeltes Titandioxid vor.

Komponente B ist in den erfindungsgemäßen Mischungen in einer Menge von 40 bis 95 Gew.-%, vorzugsweise 60 bis 90 Gew.-%, insbesondere 70 bis 85 Gew.-% enthalten. Bei Komponente B handelt es sich um eine organische Mirkopigment-Dispersion, die aus den Komponenten B1 bis B4 besteht, deren Gesamtmenge 100 Gew.-% ergibt. Komponente B1 liegt in einer Menge von 20 bis 75 Gew.-%, vorzugsweise 40 bis 60 Gew.-% vor. Es handelt sich um mindestens ein Methylen-bis-benzotriazolylphenol, in dem die Benzotriazolreste und Phenolreste jeweils unabhängig durch einen oder mehrere C_{1 - 20}-Alkylreste, C_{1 - 20}-Alkoxyreste, C_{1 - 20}-Hydroxyalkylreste, C_{6 - 12}-Arylreste, Halogen, Hydroxalgruppen oder Cyanogruppen substituiert sein können. Vorzugsweise sind nur die Phenolreste einfach oder zweifach substituiert, wobei der Substituent vorzugsweise in p-Position zur Hydroxylgruppe vorliegt. Besonders bevorzugt sind die Phenolreste durch einen p-ständigen C_{4 - 12}-Alkylrest substituiert. Insbesondere handelt es sich bei Komponente B1 um 2,2'-Methylen-bis-(6-(2H-benzotriazol-2-yl)-4-(1,1,3,3-tetramethylbutyl)phenol), wie es in der Formulierung Tinosorb^{®} M von Ciba Specialty Chemicals vorliegt.

Als Komponente B2 liegen 2 bis 15 Gew.-%, vorzugsweise 5 bis 10 Gew.-% mindestens eines Emulgators vor. Geeignete Emulgatoren sind die nachstehend angegebenen Emulgatoren. Insbesondere werden Alkylglucoside oder Alkylpolyglucoside eingesetzt. Speziell werden C_{6 - 20}-Alkylglucoside, insbesondere Decylglucoside eingesetzt.

Als Komponente B3 liegen 0,5 bis 5 Gew.-%, vorzugsweise 0,5 bis 1 Gew.-% organische Hilfsstoffe vor. Dabei handelt es sich vorzugsweise um Verdicker und organische Lösungsmittel. Als Verdicker können die nachstehend aufgeführten Verdicker, insbesondere Xanthan Gumen, eingesetzt werden. Ein Beispiel eines geeigneten organischen Lösungsmittels ist Propylenglycol. Beispielsweise liegen 0,2 Gew.-% Xanthan Gumen und 0,4 Gew.-% Propylenglycol vor.

Als verbleibende Komponente B4 liegt Wasser in einer Menge vor, so dass die Gesamtmenge der Komponenten 100 Gew.-% ergibt. Die Menge der Komponente B4 beträgt in der Regel 22,5 bis 77,5 Gew.-%, vorzugsweise 30 bis 50 Gew.-%.

Als bevorzugte Komponente B wird die von Ciba Specialty Chemicals als Tinosorb® M vertriebene Dispersion eingesetzt.

Erfindungsgemäß wird das, vorzugsweise teilchenförmige, Titandioxid der Komponente A als Formulierungshilfsmittel für die organischen Mikropigmentdispersionen B bei der Herstellung von kosmetischen OW-, WO-, PO- oder multiplen Emulsionen eingesetzt.

Insbesondere das mikrofeine Titandioxid dispergiert sich in den Formulierungen noch selbst, so dass eine mikrofeine Pigmentverteilung der organischen und anorganischen Pigmente vorliegt. Das Dispersiongleichgewicht des organischen Mikropigments wird dabei nicht beeinflusst bzw. nicht verschlechtert.

Bei gleichzeitiger Verwendung des Titandioxids mit dem organischen Mikropigment in Lichtschutzmitteln ergibt sich ein synergistischer Effekt, der über eine additive Lichtschutzwirkung bei der Komponenten weit hinausgeht.

Ein derartiger synergistischer Effekt wurde bislang nur für die Kombination von Tinosorb^{®} M mit Octylmethoxycinnamat (OMC) gefunden. Erfindungsgemäß wurde ein synergistischer Lichtschutzeffekt jetzt für die Kombination des festen W-Filters mit Titandioxid gefunden.

Damit sind die erfindungsgemäßen Mischungen besonders vorteilhaft einsetzbar bei der Herstellung und Formulierung von kosmetischen Emulsionen oder Lichtschutzmittel-Emulsionen. Die Erfindung betrifft auch ein Verfahren zur Herstellung von OW-, WO-, PO- oder multiplen Emulsionen, die organische Mikropigment-Dispersionen enthalten, wobei man eine wie vorstehend beschriebene Mischung in die OW-, WO-, PO- oder multiplen Emulsionen einbringt und nachfolgend homogenisiert.

Die Erfindung betrifft auch eine WO-Emulsion, die eine Ölphase, eine Wasserphase und, bezogen auf die gesamte Emulsion, 1 bis 50 Gew.-%, vorzugsweise 3 bis 15 Gew.-% der vorstehend beschriebenen Mischung enthält.

Bei der Herstellung der OW-, WO-, PO- oder multiplen Emulsionen kann die erfindungsgemäße Mischung direkt eingesetzt werden, oder die Komponenten A und B der erfindungsgemäßen Mischung können getrennt in die Emulsionen eingebracht werden. Es ist dabei möglich, die Komponenten A und B oder deren Mischung in eine oder mehrere der zur Herstellung der Emulsionen eingesetzten Phasen einzubringen und die einzelnen Phasen nachfolgend zu vermischen. Es ist andererseits auch erfindungsgemäß möglich, zunächst die fertige Emulsion ohne die erfindungsgemäße Mischung herzustellen und sodann die Mischung oder die Komponenten A und B in die Emulsion einzubringen.

Weitere geeignete Inhaltsstoffe der Emulsionen sind nachstehend beschrieben. Es kann auch auf die entsprechenden Offenbarungen in DE-A-197 26 184, DE-A-199 23 645, DE-A-199 23 713 und DE-A-199 33 461 verwiesen werden.

Die Emulsionen können als hochviskose Systeme wie Gele vorliegen. Die Herstellung von Emulsionen, Salben, Gelen, Liposomen für alle üblichen Bereiche, wie pharmazeutischen, kosmetischen, dermatologischen, lebensmitteltechnologischen oder waschmitteltechnologischen Bereiche ist möglich. Auch andere Anwendungsgebiete sind erfindungsgemäß zugänglich. Es kann sich dabei um Makro-, Mikro- oder Nanoemulsionen handeln. Nanoemulsionen weisen dabei Emulsionströpfchen mit einem mittleren Durchmesser im Bereich von 5 bis 1000 nm, vorzugsweise 15 bis 300 nm auf.

Die erfindungsgemäßen Mischungen können auch zur Herstellung von wässrigen Wirkstoffträger-Nanodispersionen, die mindestens einen pharmazeutischen oder kosmetischen Wirkstoff enthalten, eingesetzt werden. Als Wirkstoffirägerteiichen werden hierbei Teilchen auf Lipidbasis eingesetzt. Hierzu gehören Lipide und lipidähnliche Strukturen. Beispiele geeigneter Lipide sind die Mono-, Di- und Triglyceride der gesättigten geradkettigen Fettsäuren mit 12 bis 30 Kohlenstoffatomen, wie Laurinsäure, Myristinsäure, Palmitinsäure, Stearinsäure, Arachinsäure, Behensäure, Lignocerinsäure, Cerotinsäure, Melesinsäure, sowie deren Ester mit anderen mehrwertigen Alkoholen wie Ethylenglykol, Propylenglykol, Mannit, Sorbit, gesättigten Fettalkoholen mit 12 bis 22 Kohlenstoffatomen wie Laurylalkohol, Myrestylalkohol, Cetylalkohol, Stearylalkohol, Arachidylalkohol, Behenylalkohol, gesättigten Wachsalkoholen mit 24 bis 30 Kohlenstoffatomen wie Lignocerylalkohol, Cerylalkohol, Ceratylalkohol, Myristylalkohol. Bevorzugt sind Mono-, Di-, Triglyceride, Fettalkohole, deren Ester oder Ether, Wachse, Lipidpeptide oder Mischungen davon. Insbesondere werden synthetische Mono-, Di- und Triglyceride als Einzelsubstanzen oder in Form einer Mischung, zum Beispiel in Form eines Hartfettes, eingesetzt. Glycerintrifettsäureester sind beispielsweise Glycerintrilaurat, Glycerintrimyristat, Glycerinpalmitat, Glycerintristearat oder Glycerintribehenat. Geeignete Wachse sind beispielsweise Cetylpalmitat und Cera alba (gebleichtes Wachs, DAB 9). Als Lipide können auch Polysaccharide, Polyalkylacrylate, Polyalkylcyanoacrylate, Polyalkylvinylpyrrolidone, Acrylpolymere, Polymilchsäuren oder Polylactide eingesetzt werden.

Die Menge der Wirkstoffträgerteilchen, bezogen auf die gesamte wässrige Wirkstoffträger-Dispersion, beträgt vorzugsweise 0,1 bis 30 Gew.-%, besonders bevorzugt 1 bis 10 Gew.-%. Zusätzlich zu den Lipiden können Dispersionsstabilisatoren eingesetzt werden. Sie können beispielsweise in Mengen von 0,01 bis 10 Gew.-%, vorzugsweise 0,05 bis 5 Gew.-% eingesetzt werden. Beispiele geeigneter Substanzen sind Tenside, insbesondere ethoxylierte Sorbitanfettsäureester, Blockpolymere und Blockcopolymere (wie zum Beispiel Poloxamere und Poloxamine), Polyglycerinether und -ester, Lecithine verschiedenen Ursprungs (zum Beispiel Ei- oder Sojalecithin), chemisch modifizierte Lecithine (zum Beispiel hydriertes Lecithin) als auch Phospholipide und Sphingolipide, Mischungen von Lecithinen mit Phospholipiden, Sterine (zum Beispiel Cholesterin und Cholesterinderivate sowie Stigmasterin), Ester und Ether von Zuckern oder Zuckeralkoholen mit Fettsäuren oder Fettalkoholen (zum Beispiel Saccharosemonostearat), sterisch stabilsierende Substanzen wie Poloxamere und Poloxamine (Polyoxyethylen-Polyoxypropylen-Blockpolymere), ethoxylierte Sorbitanfettsäureester, ethoxylierte Mono- und Diglyceride, ethoxylierte Lipide und Lipoide, ethoxylierte Fettalkohole oder Fettsäuren und Ladungsstabilisatoren bzw. Ladungsträger wie zum Beispiel Dicetylphosphat, Phosphatidylglycerin sowie gesättigte und ungesättigte Fettsäuren, Natriumcholat, Natriumglykolcholat, Natriumtaurocholat oder deren Mischungen, Aminosäuren oder Peptisatoren wie Natriumcitrat (siehe J. S. Lucks, B. W. Müller, R. H. Müller, Int. J. Pharmaceutics 63, Seiten 183 bis 189 (1990)), viskositätserhöhende Stoffe wie Celluloseether und -ester (zum Beispiel Methylcellulose, Hydroxyethylcellulose, Hydroxypropylcellulose, Natriumcarboxymethylcellulose), Polyvinylderivate wie Polyvinylalkohol, Polyvinylpyrrolidon, Polyvinylacetat, Alginate, Polyacrylate (zum Beispiel Carbopol), Xanthane und Pektine.

Als wässrige Phase können Wasser, wässrige Lösungen oder Mischungen von Wasser mit wassermischbaren Flüssigkeiten wie Glycerin oder Polyethylenglycol eingesetzt werden. Weitere zusätzliche Komponenten für die wässrige Phase sind beispielsweise Mannose, Glucose, Fructose, Xylose, Trehalose, Mannit, Sorbit, Xylit oder andere Polyole wie Polyethylenglykol sowie Elektrolyte wie Natriumchlorid. Diese zusätzlichen Komponenten können in einer Menge von 0,5 bis 60, zum Beispiel 1 bis 30 Gew.-%, bezogen auf die wässrige Phase A, eingesetzt werden.

Falls gewünscht, können ferner viskositätserhöhende Stoffe oder Ladungsträger eingesetzt werden, wie Sie in EP-B-0 605 497 beschrieben sind.

Als Emulgatoren, die Lamellarstrukturen ausbilden, können natürliche oder synthetische Produkte eingesetzt werden. Auch der Einsatz von Tensidgemischen ist möglich. Beispiele geeigneter Emulgatoren sind die physiologischen Gallensalze wie Natriumcholat, Natriumdehydrocholat, Natriumdeoxycholat, Natriumglykocholat, Natriumtaurocholat.

Tierische und pflanzliche Phospholipide wie Lecithine mit ihren hydrierten Formen sowie Polypeptide wie Gelatine mit ihrem modifizierten Formen können ebenso verwendet werden.

Als synthetische grenzflächenaktive Substanzen eignen sich die Salze der Sulfobernsteinsäureester, Polyoxyethylensäurebetanester, Säurebetanester und Sorbitanether, Polyoxyethylenfettalkoholether, Polyoxyethylenstearinsäureester sowie entsprechende Mischungkondensate von Polyoxyethylen-Methpolyoxypropylenethern, ethoxylierte gesättigte Glyceride, partielle Fettsäure-Glyceride und Polyglycide. Beispiele geeigneter Tenside sind Biobase^{®} EP und Ceralution^{®} H.

Beispiele geeigneter Emulgatoren sind ferner Glycerinester, Polyglycerinester, Sorbitanester, Sorbitolester, Fettalkohole, Propylenglykolester, Alkylglucositester, Zuckerester, Lecithin, Silikoncopolymere, Wollwachs und deren Mischungen oder Derivate. Glycerinester, Polyglycerinester, Alkoxylate und Fettalkohole sowie Isoalkohole können sich beispielsweise ableiten von Rizinusfettsäure, 12-Hydroxystearinsäure, Isostearinsäure, Ölsäure, Linolsäure, Linolensäure, Stearinsäure, Myristinsäure, Laurinsäure und Caprinsäure. Neben den genannten Estern können auch Succinate, Amide oder Ethanolamide der Fettsäuren vorliegen. Als Fettsäurealkoxylate kommen insbesondere die Ethoxylate, Propoxylate oder gemischten Ethoxylate/Propoxylate in Betracht.

Zur Herstellung der erfindungsgemäßen kosmetischen Emulsionen werden in der Regel Emulgatoren verwendet. Beispiele geeigneter Emulgatoren sind Glycerinester, Polyglycerinester, Sorbitanester, Sorbitolester, Fettalkohole, Propylenglykolester, Alkylglucosidester, Zuckerester, Lecithin, Silikoncopolymere, Wollwachs und ihre Mischungen und Derivate. Glycerinester, Polyglycerinester, Alkoxylate und Fettalkohole sowie Isoalkohole können sich beispielsweise ableiten von Rhizinusfettsäure, 12-Hydroxystearinsäure, Isostearinsäure, Ölsäure, Linolsäure, Linolensäure, Stearinsäure, Myrestinsäure, Maurinsäure und Caprinsäure. Neben den genannten Estern können auch Succinate, Amide oder Ethanolamide der Fettsäuren vorliegen. Als Fettsäurealkoxylate kommen insbesondere die Ethoxylate, Propoxylate oder gemischten Ethoxylate/Propoxylate in Betracht. Ferner können Emulgatoren eingesetzt werden, die Lämelastrukturen ausbilden. Beispiele derartiger Emulgatoren sind die physiologischen Gallensalze wie Natriumcheolat, Natriumdehydrocheolat, Natriumdeoxycheolat, Natriumglycochealat, Natriumtaurochealat. Tierische und pflanzliche Phospholipide wie Lecithine mit Ihren hydrierten Formen sowie Polypeptide wie Gelatine mit ihren modifizierten Formen können ebenso verwendet werden. Als synthetische grenzflächenaktive Substanzen eignen sich die Salze der Sulfobernsteinsäureester, Polyoxiethylensäurebethanester, Säurebethanester und Sorbitanether, Polyoxiethylenfettalkoholether, Polyoxiethylenstearinsäureester sowie entsprechende Mischungskondensate von Polyoxiethylen-methpolyoxipropylenethern, ethoxylierte gesättigte Glyceride, partielle Fettsäure-Glyceride und Polyglycide. Beispiele geeigneter Tenside sind Biobase^{®} EP und Ceralution^{®} H.

Lipide und Emulgatoren werden vorzugsweise in einem Gewichtsverhältnis von 50: 1 bis 2: 1, vorzugsweise 15:1 bis 30:1 eingesetzt.

Die zusätzlichen pharmazeutischen, kosmetischen und/oder lebensmitteltechnologischen Wirkstoffe werden vorzugsweise in einer Menge von 0,1 bis 80 Gew.-%, besonders bevorzugt 1 bis 10 Gew.-% eingesetzt.

Bei den weiteren Wirkstoffen handelt es sich gemäß einer Ausführungsform der Erfindung um zusätzliche Lichtschutzfilter. Diese können als organische Lichtschutzfilter bei Raumtemperatur (25°C) in flüssiger oder fester Form vorliegen. Geeignete Lichtschutzfilter (UV-Filter) sind beispielsweise Verbindungen auf Basis von Benzophenon, Diphenylcyanacrylat oder p-Aminobenzoesäure. Konkrete Beispiele sind (INCI- oder CTFA-Bezeichnungen) Benzophenone-3, Benzophenone-4, Benzophenone-2, Benzophenone-6, Benzophenone-9, Benzophenone-1, Benzophenone-11, Etocrylene, Octocrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxydibenzoylmethane, Ethylhexyl Salicylate, Homosalate sowie 2-Hydroxy-4-methoxybenzophenon-5-sulfonsäure und 2,4,6-Trianilino-p-(carbo-2'-ethylhexyl-1'-oxi)-1,3,5-triazin.

Weitere organische Lichtschutzfilter sind Octyltriazone, Avobenzone, Octylmethoxycinnamate, Octylsalicylate, Benzotriazole und Triazine.

Weitere mögliche Inhaltstoffe der Emulsionen sind zusätzliche hydrophil beschichtete Mikropigmente, Elektrolyte, Glycerin, Polyethylenglykol, Propylenglykol, Bariumsulfat, Alkohole, Wachse, Metallseifen, Magnesiumstearat, Vaseline oder andere Inhaltsstoffe. Beispielsweise können weiterhin Parfums, Parfumöle oder Parfumaromen zugesetzt werden. Geeignete kosmetische Wirkstoffe sind beispielsweise Polyphenole und davon abgeleitete Verbindungen. Geeignete Vitamine sind Retinol, Tocopherol, Ascorbinsäure, Riboflavin und Pyridoxin.
Mit der erfindungsgemäßen Mischung können alle bekannten und geeigneten Wasser-in-Öl-Emulsionen oder Öl-in-Wasser-Emulsionen hergestellt werden. Dazu können die nach den beschriebenen Emulgatoren und weiteren Inhaltsstoffe eingesetzt werden. Ferner ist die Herstellung von Polyol-in-Öl-Emulsionen möglich. Hierbei können beliebige geeignete Polyole eingesetzt werden.

In den Emulsionen können die Anteile der zwei Hauptphasen in weiten Bereichen variiert werden. Beispielsweise liegen 5 bis 95 Gew.-%, vorzugsweise 10 bis 90 Gew.-%, insbesondere 20 bis 80 Gew.-% der jeweiligen Phasen vor, wobei die Gesamtmenge 100 Gew.-% ergibt.

Die beschriebene P/O-Emulsion kann auch in Wasser oder eine Wasser-in-Öl-Emulsion emulgiert werden. Dabei resultiert eine Polyol-in-Öl-in Wasser-Emulsion (P/O/W-Emulsion), die mindestens eine beschriebene Emulsion und zusätzlich mindestens eine wässrige Phase enthält. Derartige multiple Emulsionen können im Aufbau den in DE-A-43 41 113 beschriebenen Emulsionen entsprechen.

Beim Einbringen der P/O-Emulsion in Wasser oder wässrige Systeme kann das Gewichtsverhältnis der einzelnen Phasen in weiten Bereichen variiert werden. Vorzugsweise beträgt in der letztendlich erhaltenen P/O/W-Emulsion der Gewichtsanteil der P/O-Emulsion 0,01 bis 80 Gew.-%, besonders bevorzugt 0,1 bis 70 Gew.-%, insbesondere 1 bis 30 Gew.-%, bezogen auf die gesamte P/O/W-Emulsion.

Beim Einbringen der P/O-Emulsion in eine O/W-Emulsion beträgt der Anteil der P/O-Emulsion vorzugsweise 0,01 bis 60 Gew.-%, besonders bevorzugt 0,1 bis 40 Gew.-%, insbesondere 1 bis 30 Gew.-%, bezogen auf die letztendlich erhaltene P/O/W-Emulsion. In der O/W-Emulsion, die hierzu verwendet wird, beträgt der Ölanteil vorzugsweise 1 bis 80 Gew.-%, besonders bevorzugt 1 bis 30 Gew.-%, bezogen auf die eingesetzte O/W-Emulsion. Anstelle einer P/O-Emulsion kann auch eine W/O-Emulsion eingebracht werden, was zu einer W/O/W-Emulsion führt. Die einzelnen Phasen der Emulsionen können noch übliche für die einzelnen Phasen bekannte Inhaltsstoffe aufweisen. Beispielsweise können die einzelnen Phasen weitere in diesen Phasen lösliche pharmazeutische oder kosmetische Wirkstoffe enthalten. Die wässrige Phase kann beispielsweise zusätzliche organische lösliche Lichtschutzfilter, zusätzliches hydrophil gecoatetes Micropigment, Elektrolyte, Alkohole usw. enthalten. Einzelne oder alle der Phasen können zudem zusätzliche Feststoffe enthalten, die vorzugsweise ausgewählt sind aus Pigmenten oder Micropigmenten, Mikrosphären, Silikagel und ähnlichen Stoffen. Die Ölphase kann beispielsweise zusätzlich organisch modifizierte Tonmineralien, hydrophob gecoatete (Micro)Pigmente, organische öllösliche Lichtschutzfilter, öllösliche kosmetische Wirkstoffe, Wachse, Metallseifen wie Magnesiumstearat, Vaseline oder Gemische davon enthalten. Als zusätzliche (Micro)Pigmente können Titandioxid, Zinkoxid und Bariumsulfat sowie Wollastonit, Kaolin, Talk, Al₂O₃, Bismutoxidchlorid, micronisiertes Polyethylen, Glimmer, Ultramarin, Eosinfarben, Azofarbstoffe, genannt werden.

Die Wasserphase kann darüber hinaus Glycerin, Polyethylenglykol, Propylenglykol, Ethylenglykol und ähnliche Verbindungen sowie Derivate davon enthalten.

Die Verwendung von üblichen Hilfs- und Zusatzstoffen in den Emulsionen ist dem Fachmann bekannt.

Als wässrige Phase können Wasser, wässrige Lösungen oder Mischungen von Wasser mit wassermischbaren Flüssigkeiten wie Glycerin oder Polyethylenglykol eingesetzt werden. Ferner können in der wässrigen Phase Elektrolyte wie Natriumchlorid enthalten sein. Falls gewünscht, können ferner viskositätserhöhende Stoffe oder Ladungsträger eingesetzt werden, wie sie in der EP-B-0605 497 beschrieben sind.

Die Erfindung wird durch die nachstehenden Beispiele näher erläutert.

### Beispiele

In den folgenden Beispielen wird die Pigmentdispersion Tinosorb^{®} M der Ciba Specialty Chemicals eingesetzt, wie sie in der vorstehenden Beschreibung mit den Inhaltsstoffen erläutert ist.

### Beispiel 1

Die organische Pigmentdispersion Tinosorb^{®} M wurde mit unterschiedlichen feinteiligen Titandioxid-Pulvern vermischt. Sodann wurde die Teilchengrößenverteilung durch Laserlichtstreuung bestimmt. In der nachstehenden Tabelle sind die Zusammensetzungen und die erhaltenen mittleren Teilchengrößen aufgeführt.

UV-Titan X11 ist ein mit Aluminiumoxid modifiziertes Titandioxid mit einer Teilchengröße von etwa 14 nm. Die spezifische Oberfläche beträgt etwa 100 m²/g.

**Tabelle 1**

| Partikelgrößenanalysen verschiedener wässriger Pigmentdispersionen | | | |
|---|---|---|---|
| Pigment in Wasser | mittlerer | Anteil der | spezifische |
| dispergiert | Partikeldurchmesser | Partikel < 1 µm | Partikeloberfläche |
| | median [µm] | [%] | [cm2/cm3] |
| UV Titan M212 | 0,37 | 95,2 | 125632 |
| UV Titan X111 | 0,36 | 77,5 | 16432 |
| Tinosob M | 0,28 | 100 | 215085 |
| Tinosorb M + UV | 0,37 | 95,2 | 125632 |
| Titan M212 | | | |
| Tinosorb M + UV | 0,29 | 99 | 132053 |
| Titan X111 | | | |

Aus den Ergebnissen der Tabelle geht hervor, dass beim Vermischen von Tinosorb^{®} M mit den Titandioxid-Teilchen sich ein Mittelwert der Teilchengrößenverteilung einstellte, der in der Mitte der Mittelwerte beider Teilchengrößenverteilungen für Tinosorb^{®} M und Titandioxid-Pigment allein erhalten wurde.

Die Breite der Verteilungen änderte sich durch die Kombination von Tinosorb^{®} M und Titandioxid-Teilchen nicht.

### Beispiel 2 (Vergleich)

### OW-Sonnenschutzmittel, formuliert mit TiO₂ oder Tinosorb^{®} M

| **Handelsname** | **Hersteller** | **CTFA/INCI** | **2a** | **2b** |
|---|---|---|---|---|
| | | | **[Gew.-%]** | **[Gew.-%]** |
| **Phase A** | | | | |
| Tegocare 450 | Golschmidt | Polyglyceryl-3 methyl glucose distear | 5,00 | 5,00 |
| Miglyol 812 N | Sasol | Caprylic/capric triglyceride | 21,00 | 21,00 |

| **Phase B** | | | | |
|---|---|---|---|---|
| demin. Water | - | Aqua | 67,70 | 55,70 |
| Ceralution F | Sasol | Sodium lauroyl lactylate, sodium dico | 1,00 | 1,00 |
| Keltrol | NutraSweet | Xanthan gum | 0,30 | 0,30 |

| **Phase C** | | | | |
|---|---|---|---|---|
| UV-Titan X111 | Kemira | Titanium dioxide, alumina | **4,00** | 0,00 |
| Tinosorb M | Ciba | Methylene Bis-Benzotriazolyl Tetram | 0,00 | **16,00** |
| Phase D | | | | |
| Phenonip | Nipa | Phenoxyathanol, methylparaben, eth | 1,00 | 1,00 |
| **total:** | | | **100,00** | **100,00** |

Zur Herstellung wurden die Phasen A und B getrennt auf 70 °C erwärmt. Phase A wurde zu Phase B gegeben, und es wurde homogenisiert. Sodann wurde auf 30 °C abgekühlt, Phase C wurde zur gemischten Phase AB gegeben, und es wurde homogenisiert. Abschließend würde Phase D zur Phase ABC gegeben, und es wurde langsam gerührt.

Beide Zusammensetzungen wurden einem Stabilitätstest unterzogen. Die Ergebnisse sind in der nachstehenden Aufstellung zusammengefasst.

| **Stabilitätstest** | **2 a** | **2 b** |
|---|---|---|
| **RT** | | |
| 3 Tage | 1 | 1 |
| 1 Woche | 1 | 1 |
| 2 Wochen | 1 | 1 |
| | | |

| **40 °C** | | |
|---|---|---|
| 1 Tag/3 Tage | 1 | 1 |
| 5 Tage | 1 | 1 |
| 1 Woche | 1 | 4/O 5 % |
| | | |

| **50 °C** | | |
|---|---|---|
| 1 Tag/3 Tage | 1 | 1 |
| 5 Tage | 1 | 1 |
| 1 Woche | 1 | 5 |
| | | |

| **-18 °C/40 °C** | | |
|---|---|---|
| 1 x | 1 | 1 |
| 2 x | 1 | 1 |
| 3 x | 1 | 3/O |
| 4 x | 1 | 3/O |
| 5 x | | 3/O |

| | | |
|---|---|---|
| Beobachtung: 1 = OK 2 = inhomogen 3/W = sichtbare Wasserabscheidung 3/O = sichtbare Ölabscheidung 4/W = messbare Wasserabscheidung 4/O = messbare Ölabscheidung 5 = totale Separation | | |

### Beispiel 3

### OW-Sonnenschutzmittel, formuliert mit einer Mischung aus TiO₂ und Tinosorb® M

| **Handelsname** | **Hersteller** | **CTFA/INCI** | **3 [Gew.-%]** |
|---|---|---|---|
| **Phase A** | | | |
| Ceralution H | Sasol | Sodium lauroyl lactylate, sodium dicocoylethylendiamine PEG-15 sulfate | 4,00 |
| Miglyol 812 N | Hüls | Caprylic/capric triglyceride | 21,00 |

| **Phase B** | | | |
|---|---|---|---|
| demin. water | | | 40,90 |
| Ceralution F | Sasol | Sodium lauroyl lactylate, sodium dicocoylethylenediamine PEG-15 sulfate | 1,00 |
| Keltrol | Kelco | Xanthan gum | 0,30 |

| **Phase C** | | | |
|---|---|---|---|
| Tinosorb M | Ciba | Methylene Bis-Benzotriazolyl Tetramethylbutylphenol | 16,00 |
| UV-Titan X111 | Kemira | Titanium dioxide, alumina | 4,00 |

| **Phase D** | | | |
|---|---|---|---|
| Carbopol ETD 2050 (3 %) | BF Goodrich | Carbomer | 10,00 |

| **Phase E** | | | |
|---|---|---|---|
| 10 % NaOH | | | 1,80 |

| **Phase F** | | | |
|---|---|---|---|
| Phenonip | Nipa | Phenoxyethanol, methylparaben, ethylparaben, butylparaben, propylparaben, isobutylparaben | 1,00 |
| **total:** | | | **100,00** |

Die Herstellung erfolgte durch getrenntes Aufheizen der Phasen A und B auf 70 °C, nachfolgendes Zugeben der Phase A zur Phase B, und Homogenisieren. Sodann wurde auf 30 °C abgekühlt und Phase C (X111 vordispergiert in Tinosorb^{®} M) zur Phase AB gegeben. Nachfolgend wurde homogenisiert. Sodann wurde Phase D unter Rühren eingetragen, und es wurde mit Phase E neutralisiert. Sodann wurde Phase F unter Rühren mit einem Spatel eingetragen.
Die Zusammensetzung wies einen in vivo-Sonnenschutzfaktor von 18,7 auf.

Mit den Formulierungen aus Beispiel 2 und Beispiel 3 konnte im Rahmen von Lagertests gezeigt werden, dass die Einarbeitung von Tinosorb^{®} M in OW-Formulierungen zu Instabilitäten führt. Diese Instabilität ist auf die Wirkung des Tinosorb^{®} M-Dispergierhilfsmittels (APG) zurückzuführen. APG wird als hydrophiles Tensid in das flüssigkristalline Gelnetzwerk der OW-Emulsion eingebaut und destabilisiert dieses. Daher erwies sich auch die Formulierung gemäß Beispiel 2b, die nur Tinosorb^{®} M enthielt, als nicht lagerfähig.

Im Vergleich dazu führte die Einarbeitung des anorganischen Pigments und die Einarbeitung des Gemisches aus Tinosorb^{®} M und anorganischem Pigment zu keiner Destabilisierung, siehe die Beispiele 2a und 3.

Die Stabilität der Rezeptur aus Beispiel 3 wurde für 3 Monate bei 50 °C und 5 Tan-Gefrierzyklen getestet. Für diesen Zeitraum und die Anzahl der Tan-Gefrierzyklen blieb die Stabilität der Emulsion erhalten.

### Beispiel 4

### WO-Sonnenschutzmittel, formuliert mit einer Mischung aus TiO₂ und Tinosorb^{®} M

| **Handelsname** | **Hersteller** | **CTFA/INCI** | **[Gew.-%]** |
|---|---|---|---|
| **Phase A** | | | |
| Abil WE | Golschmidt | Polyglyceryl-4 isostearate, cetyl dimethicone copolyl, hexyl laurate | 5,00 |
| Amisol 110 | Lucas Meyer GmbH | Lecithin, oleamide DEA, soybean oil | 1,25 |
| Finsolv TN | | | 12,00 |
| Arlamol HD | Uniqema | Heptanlethylnonane | 5,00 |
| Cetiol SN | Cognis | Cetearyl isononanoate | 12,00 |
| Vitamin E Acetat | Roche | Tocopheryl acetate | 1,00 |

| **Phase B** | | | |
|---|---|---|---|
| demin. water | | | 41,35 |
| Propylenglycol | Overlack | Propylene glycol | 2,00 |
| Natriumchlorid | Merck | Sodium chloride | |

| **Phase C** | | | |
|---|---|---|---|
| UV-Titan X111 | Kemira | | 4,00 |
| TINOSORB M | Ciba | Methylene Bis-Benzotriazolyl Tetramethylbutylphenol | 16,00 |
| **total:** | | | **100,00** |

Zur Herstellung wurde Phase B zu Phase A gegeben, und es wurde homogenisiert. Sodann wurde Phase C zur Phase AB gegeben, und es wurde homogenisiert.

Das Lichtschutzmittel wies einen in vivo-Sonnenschutzfaktor von 13,4 auf.

### Beispiel 5

### WO-Sonnenschutzmittel, formuliert mit TiO₂/Tinosorb^{®} M

| Handelsname | 5a | 5b | 5c |
|---|---|---|---|
| | Gew.-% | (Vergl.) | (Vergl.) |
| **Phase A** | HS | BB | BB |
| Abil WE 09 | 5.00 | 5.00 | 5.00 |
| Amisol 110 | 1.25 | 1.25 | 1.25 |
| Finsolv TN | 12.00 | 12.00 | 12.00 |
| Arlamol HD | 5.00 | 5.00 | 5.00 |
| Cetiol SN | 12.00 | 12.00 | 12.00 |
| Vitamin E Acetat | 1.00 | 1.00 | 1.00 |

| **Phase B** | | | |
|---|---|---|---|
| demin. Wasser | 41.35 | 57.35 | 45.35 |
| Propylenglycol | 2.00 | 2.00 | 2.00 |
| Tinosorb M | **16.00** | 0.00 | **16.00** |
| UV-Titan X111 | **4.00** | **4.00** | 0.00 |
| Natriumchlorid | 0.40 | 0.40 | 0.40 |
| **total:** | **100.00** | **100.00** | **100.00** |

Die Herstellung erfolgte durch Dispergieren des Pigments der Phase B in Tinosorb^{®} M und Verdünnung mit Wasser. Sodann wurde Phase B langsam zu Phase A gegeben, und es wurde für eine Minute homogenisiert.

Für Beispiel 5 a ergab sich ein in vitro-Sonnenschutzfaktor von 21,9.

Der Stabilitätstest entsprechend Beispiel 2 ergab folgende Ergebnisse:

| | **5a** | **5b** | **5c** |
|---|---|---|---|
| **RT** | | | |
| 1 Tag | 1 | 1 | 1 |
| 3 Tage | 1 | 1 | 1 |
| 1 Woche | 1 | 1 | 1 |
| 2 Wochen | 1 | 1 | 1 |
| 3 Wochen | 1 | | |
| 1 Monate | 1 | | |
| 2 Monate | 1 | | |
| 3 Monate | 1 | | |
| | | | |

| **40 °C** | | | |
|---|---|---|---|
| 1 Tag | 1 | 1 | 4/W 10 % |
| 3 Tage | 1 | 1 | 4/W 10 % |
| 1 Woche | 1 | 1 | - |
| 2 Wochen | 1 | 1 | - |
| 3 Wochen | 4/O 10 % | | - |
| | | | |

| **50 °C** | | | |
|---|---|---|---|
| 1 Tag | 1 | 1 | 4/W 30 % |
| 3 Tage | 1 | 1 | - |
| 1 Woche | 1 | 1 | - |
| 2 Wochen | 3/O | 1 | - |
| 3 Wochen | 3/O | | - |
| 4 Wochen | 3/O | | - |
| 2 Monate | 4/O 20 % | | - |
| 3 Monate | 4/O 20 % | | - |
| | | | |

| **-18 °C/40 °C** | | | |
|---|---|---|---|
| 1 x | 1 | 1 | 1 |
| 2 x | 1 | 1 | 4/W5% |
| 3 x | 1 | 1 | 4/W5% |
| 4 x | 3/O | 1 | 4W 5 % |

## Patentansprüche

1. Mischung, bestehend aus
a) 10 bis 40 Gew.-% teilchenförmigem Titandioxid mit einer mittleren Teilchengröße im Bereich von 5 bis 500 nm, auf das organische und/oder anorganische Hilfsmittel aufgebracht sein können, als Komponente A,
b) 60 bis 90 Gew.-% einer organischen Mikropigment-Dispersion als Komponente B, bestehend aus
b1) 20 bis 75 Gew.-% mindestens eines Methylen-bis-benzotriazolylphenols, in dem die Benzotriazolylreste und Phenolreste jeweils unabhängig durch einen oder mehrere C₁₋₂₀-Alkylreste, C₁₋₂₀-Alkoxyreste, C₁₋₂₀-Hydroxyalkylreste, C₆₋₁₂-Arylreste, Halogen, Hydroxylgruppen oder Cyanogruppen substituiert sein können, als organisches Mikropigment B1,
b2) 2 bis 15 Gew.-% mindestens eines Emulgators B2,
b3) 0,5 bis 5 Gew.-% organischen Hilfsstoffen B3,
b4) 22,5 bis 77,5 Gew.-% Wasser als Komponente B4,
wobei die Gesamtmenge der Komponenten B1 bis B4 100 Gew.-% ergibt,
wobei bis zu 20 Gew.-% der Mischung durch andere Inhaltsstoffe ersetzt sein können, erhältlich durch Zusatz der Komponente A zur Komponente B.

2. Mischung nach Anspruch 1, **dadurch gekennzeichnet, dass** das organische Mikropigment ein Methylen-bis-benzotriazolyl (C₄₋₁₂-alkylphenol) ist.

3. Mischung nach Anspruch 1 oder 2, **dadurch gekennzeichnet, dass** Komponente B aus 40 bis 60 Gew.-% der Komponente B1, 5 bis 10 Gew.-% der Komponente B2, 0,5 bis 1 Gew.-% Verdickern und organischen Lösungsmitteln als Komponente B3 und 30 bis 50 Gew.-% der Komponente B4 besteht, wobei die Gesamtmenge der Komponenten B1 bis B4 100 Gew.-% ergibt.

4. Verwendung einer Mischung gemäß einem der Ansprüche 1 bis 3 zur Herstellung von kosmetischen OW-, WO-, PO- oder multiplen Emulsionen.

5. Verwendung von teilchenförmigem Titandioxid mit einer mittleren Teilchengröße im Bereich von 5 bis 500 nm, auf das organische und/oder anorganische Hilfsmittel aufgebracht sein können, zur Verbesserung der Formulierbarkeit von organischen Mikropigment-Dispersionen B, wie sie in einem der Ansprüche 1 bis 3 definiert sind, bei der Herstellung von kosmetischen OW-, WO-, PO- oder multiplen Emulsionen.

6. Verfahren zur Herstellung von OW-, WO-, PO- oder multiplen Emulsionen, die Methylen-bis-benzotriazolylphenole enthalten, in denen die Benzotriazolreste und Phenolreste jeweils unabhängig durch einen oder mehrere C₁₋₂₀-Alkylreste, C₁₋₂₀-Alkoxyreste, C₁₋₂₀-Hydroxylalkylreste, C₆₋₁₂-Arylreste, Halogen, Hydroxylgruppen oder Cyanogruppen substituiert sein können, enthalten, **dadurch gekennzeichnet, dass** man eine Mischung gemäß einem der Ansprüche 1 bis 3 in die OW-, WO-, PO- oder multiplen Emulsionen einbringt und nachfolgend homogenisiert.

7. Verfahren nach Anspruch 6, **dadurch gekennzeichnet, dass** die Emulsion eine kosmetische Emulsion oder Lichtschutzmittel-Emulsion ist.

## Claims

1. A mixture consisting of
a) from 10 to 40% by weight of particulate titanium dioxide having an average particle size in the range from 5 to 500 nm, onto which titanium dioxide organic and/or inorganic auxiliaries can be applied, as component A,
b) from 60 to 90% by weight of an organic micropigment dispersion as component B consisting of
b1) from 20 to 75% by weight of at least one methylene bis-benzotriazolylphenol, wherein the benzotriazolyl residues and phenol residues each can be independently substituted by one or more C₁₋₂₀ alkyl residues, C₁₋₂₀ alkoxy residues, C₁₋₂₀ hydroxyalkyl residues, C₆₋₁₂ aryl residues, halogen, hydroxyl groups or cyano groups, as organic micropigment B1,
b2) from 2 to 15% by weight of at least one emulsifier B2,
b3) from 0.5 to 5% by weight of organic auxiliaries B3,
b4) from 22.5 to 77.5% by weight of water as component B4,
wherein the total amount of components B1 to B4 is 100% by weight,
wherein up to 20% by weight of the mixture can be replaced by other constituents, obtainable by adding component A to component B.

2. The mixture according to claim 1, **characterized in that** the organic micropigment is a methylene bis-benzotriazolyl (C₄₋₁₂ alkylphenol).

3. The mixture according to claim 1 or 2, **characterized in that** component B consists of from 40 to 60% by weight of component B1, from 5 to 10% by weight of component B2, from 0.5 to 1 % by weight of thickeners and organic solvents as component B3, and from 30 to 50% by weight of component B4, wherein the total amount of components B1 to B4 is 100% by weight.

4. Use of a mixture according to any one of claims 1 to 3 for producing cosmetic OW-, WO-, PO- or multiple emulsions.

5. Use of particulate titanium dioxide having an average particle size of from 5 to 500 nm, onto which titanium dioxide organic and/or inorganic auxiliaries can be applied, for improvement of formulability of organic micropigment dispersions B as defined in any one of claims 1 to 3, for the preparation of cosmetic OW-, WO-, PO- or multiple emulsions.

6. A method for the preparation of OW-, WO-, PO- or multiple emulsions containing methylene bis-benzotriazolylphenols, wherein the benzotriazole residues and phenol residues each can be independently substituted by one or more C₁₋₂₀ alkyl residues, C₁₋₂₀ alkoxy residues, C₁₋₂₀ hydroxyalkyl residues, C₆₋₁₂ aryl residues, halogen, hydroxyl groups or cyano groups, **characterized in that** a mixture according to any one of claims 1 to 3 is added to the OW-, WO-, PO- or multiple emulsions, followed by homogenizing.

7. The method according to claim 6, **characterized in that** the emulsion is a cosmetic emulsion or sunscreen emulsion.

## Revendications

1. Mélange constitué
a) de 10 à 40 % en poids de dioxyde de titane particulaire, ayant une granulométrie moyenne comprise dans la plage de 5 à 500 nm, sur lequel peuvent être appliqués des agents auxiliaires organiques et/ou inorganiques, et servant de Composant A,
b) de 60 à 90 % en poids d'une dispersion micropigmentaire organique servant de Composant B, constituée
b1) de 20 à 75 % en poids d'au moins un méthylène-bisbenzotriazolylphénol, dans lequel les radicaux benzotriazolyle et les radicaux phénol peuvent chacun être substitués, d'une manière indépendante, par un ou plusieurs radicaux alkyle en C₁₋₂₀, alcoxy en C₁₋₂₀, hydroxyalkyle en C₁₋₂₀, aryle en C₆₋₁₂, halogéno, ou un ou plusieurs groupes hydroxyle ou cyano, servant de micropigment organique B1,
b2) de 2 à 15 % en poids d'au moins un émulsifiant B2,
b3) de 0,5 à 5 % en poids d'adjuvants organiques B3,
b4) de 22,5 à 77,5 % en poids d'eau, servant de Composant B4,
la quantité totale des Composants B1 à B4 faisant 100 % en poids,
où jusqu'à 20 % en poids du mélange peuvent être remplacés par d'autres constituants, et pouvant être obtenu par addition du Composant A au Composant B.

2. Mélange selon la revendication 1, **caractérisé en ce que** le micropigment organique est un méthylène-bisbenzotriazolyl ((alkyle en C₁₋₁₂)phénol).

3. Mélange selon la revendication 1 ou 2, **caractérisé en ce que** le Composant B est constitué de 40 à 60 % en poids du Composant B1, de 5 à 10 % en poids du Composant B2, de 0,5 à 1 % en poids d'épaississants et de solvants organiques, servant de Composant 83, et de 30 à 50 % en poids du Composant B4, où la quantité totale des Composants B1 à B4 fait 100 % en poids.

4. Utilisation d'un mélange selon l'une des revendications 1 à 3 pour préparer des émulsions cosmétiques OW (de type aqueux), WO (de type huileux), PO (polyol dans l'huile) ou multiples.

5. Utilisation de dioxyde de titane particulaire ayant une granulométrie moyenne comprise dans la plage de 5 à 500 nm, sur lequel des adjuvants organiques et/ou inorganiques peuvent être appliqués, pour améliorer l'aptitude à la formulation de dispersions B de micropigments organiques, telles que définies dans l'une des revendications 1 à 3, lors de la préparation d'émulsions cosmétiques OW, WO, PO ou multiples.

6. Procédé de préparation d'émulsions OW, WO, PO ou multiples, qui contiennent des méthylène-bisbenzotriazolylphénols, dans lesquelles les radicaux benzotriazole et les radicaux phénol peuvent chacun être substitués d'une manière indépendante par un ou plusieurs radicaux alkyle en C₁₋₂₀, alcoxy en C₁₋₂₀, hydroxyalkyle en C₁₋₂₀, aryle en C₆₋₁₂, halogéno, ou un ou plusieurs groupes hydroxyle ou cyano, **caractérisé en ce qu'**on incorpore un mélange selon l'une des revendications 1 à 3 dans les émulsions OW, WO, PO ou multiples, puis on homogénéise.

7. Procédé selon la revendication 6, **caractérisé en ce que** l'émulsion est une émulsion cosmétique ou une émulsion d'un agent photoprotecteur.
